# EUROPÄISCHE PATENTSCHRIFT

(11) **EP 3 390 340 B1**
(45) Veröffentlichungstag und Bekanntmachung des Hinweises auf die Patenterteilung: **24.06.2020**
(21) Anmeldenummer: 16823159.5
(22) Anmeldetag: 16.12.2016
(51) Int. Cl.: C07C 67/31, C07C 407/00, C07D 301/19, C07D 303/14, C07C 51/367, C07C 69/67, C07C 59/105

(54) **VERFAHREN ZUR HERSTELLUNG EINES POLYOLS**
METHOD FOR PRODUCING A POLYOL
PROCÉDÉ DE PRÉPARATION D'UN POLYOL

(30) Priorität: 17.12.2015 DE 102015016428; 08.03.2016 DE 102016002813
(43) Veröffentlichungstag der Anmeldung: 24.10.2018
(73) Patentinhaber: b2bond GmbH, 82152 Planegg (DE)
(72) Erfinder: UNVERFERTH, Maike, 68219 Mannheim (DE); MEIER, Michael, 76751 Jockgrim (DE)
(74) Vertreter: Müller-Boré & Partner Patentanwälte PartG mbB
(86) Internationale Anmeldenummer: PCT/EP2016/002122
(87) Internationale Veröffentlichungsnummer: WO 2017/102087

(56) Entgegenhaltungen:
- HAMBERG ET AL: "Vanadium-catalyzed transformation of 13(S)-hydroperoxy-9(Z), 11(E)-octadecadienoic acid: Structural studies on epoxy alcohols and trihydroxy acids", CHEMISTRY AND PHYSICS OF LIPIDS, LIMERICK, IR, Bd. 143, Nr. 1, 1. Januar 1987 (1987-01-01), Seiten 55-67, XP023389379, ISSN: 0009-3084, DOI: 10.1016/0009-3084(87)90017-X [gefunden am 1987-01-01]
- ROSS, J.; GEBHART, A. I.; GERECHT, J. F.: "The Autoxidation of Methyl Oleate", JOURNAL OF THE AMERICAN CHEMICAL SOCIETY, Bd. 71, 31. Dezember 1949 (1949-12-31), Seiten 282-286, XP002767969,

## Beschreibung

Die vorliegende Erfindung betrifft ein Verfahren zur Herstellung eines Polyols aus einer ungesättigten Fettsäure mit einer oder mehreren Doppelbindungen oder eines Polyols aus einem Fettsäurederivat, das mindestens einen ungesättigten Fettsäurerest mit einer oder mehreren Doppelbindungen aufweist.

In der Industrie werden Polyole auf Basis von Fetten und Ölen vorwiegend für die Synthese von Polyurethanen verwendet. Hierbei werden u.a. ungesättigte Triglyceride verwendet, deren Doppelbindungen epoxidiert und anschließend nukleophil z.B. mit Wasser oder Alkohol geöffnet werden, wodurch pro Doppelbindung des ungesättigten Triglycerids maximal 2 Alkoholfunktionalitäten (Hydroxylgruppen) erhalten werden.

Um höhere Hydroxylfunktionalitäten zu erhalten, musste die Öffnung des Epoxidringes entweder durch polyfunktionelle Reagenzien wie z.B. Glycerin mit Schwefelsäure als Katalysator erfolgen oder es musste von Rizinusöl ausgegangen werden, dessen Fettsäuren teilweise sowohl eine Doppelbindung als auch eine Hydroxylgruppe enthalten. Bei Verwendung von Rizinusöl kann ein Polyol mit einer großen Anzahl von Hydroxylfunktionalitäten lediglich aufgrund der bereits vorliegenden Hydroxylgruppe der Rizinolsäure, dem Hauptbestandteil von Rizinusöl, hergestellt werden. Für die Herstellung von Trihydroxystearinsäuren ging man ebenso hauptsächlich von Rizinolsäure aus. Für die Herstellung von Polyolen mit einer großen Anzahl von Hydroxylfunktionalitäten war man somit bisher weitestgehend auf einen bestimmten Ausgangsstoff oder auf den Einsatz von polyfunktionellen Reagenzien beschränkt.

Durch Epoxidierung von Rizinolsäure und anschließender Ringöffnung mit Wasser erhält man 9,10,12-Trihydroxystearinsäure, die beispielsweise in Deodorants Anwendung findet. Trihydroxystearinsäuren kommen in der Natur als Bestandteil von Pflanzen vor. 9,10,18-Trihydroxystearinsäure ist beispielsweise ein Bestandteil der beiden Biopolymere Suberin und Cutin.

Bisher war kein Verfahren bekannt, bei dem beispielsweise aus einem Triglycerid mit drei einfach ungesättigten Fettsäureresten ohne OH-Gruppen (oder anderen funktionellen Gruppen, die OH-Gruppen bilden können, wie bspw. Epoxygruppen) ein Polyol mit mehr als 6 Hydroxylfunktionalitäten hergestellt werden kann, bzw. bei dem pro Doppelbindung eines eingesetzten Triglycerids mehr als zwei Hydroxylgruppen erhalten werden können, ohne polyfunktionelle Reagenzien zu verwenden.

Mats Hamberg beschreibt eine Vanadium-katalysierte Umwandlung von 13(S)-Hydroperoxy-9(Z),11(E)-octadecadiensäure und insbesondere Strukturstudien an Epoxyalkoholen und Trihydroxysäuren (in Chemistry and Physics of Lipids, Bd. 43, Nr. 1, 1987, S. 55-67).

Ross et al., beschreiben die Autooxidation von Methyloleat (in J. Am. Chem. Soc., Bd. 71, Nr. 1, S. 282-286).

Somit liegt der vorliegenden Erfindung die Aufgabe zugrunde, ein Verfahren bereitzustellen, welches es ermöglicht, ein Polyol mit einer großen Anzahl an Hydroxylgruppen herzustellen.

Diese Aufgabe wird durch die in den Ansprüchen gekennzeichneten Ausführungsformen gelöst.

Insbesondere betrifft die vorliegende Erfindung ein Verfahren zur Herstellung eines Polyols aus einer ungesättigten Fettsäure mit einer oder mehreren Doppelbindungen oder eines Polyols aus einem Fettsäurederivat, ausgewählt aus Fettsäureester, Fettether, Fettalkohole, Fettsäureamide, Fettsäurechloride, Fettamine und Fettsäureanhydride, das mindestens einen ungesättigten Fettsäurerest mit einer oder mehreren Doppelbindungen aufweist, umfassend die Schritte
(a) Peroxidieren einer Allyl-Einheit der ungesättigten Fettsäure oder des ungesättigten Fettsäurederivats unter Bildung einer Allylhydroperoxid-Einheit, wobei anschließend die Allylhydroperoxid-Einheit zu einer Epoxyalkohol-Einheit umgesetzt wird, und
(b) Öffnen der Epoxygruppe der entstandenen Epoxyalkohol-Einheit durch Hydrolyse unter Bildung des Polyols,
wobei Schritt (a) in Gegenwart eines Übergangsmetallkatalysators erfolgt,
die Peroxidation der Allyl-Einheit in Schritt (a) eine Photoperoxidation in Gegenwart eines Peroxidationsmittels und eines Photosensibilisators ist, wobei Singulettsauerstoff als Peroxidationsmittel verwendet wird, und wobei
die Katalyse in Schritt (a) *in situ* erfolgt.

Erfindungsgemäß wird unter Peroxidieren einer Allyl-Einheit im Schritt (a) verstanden, dass mindestens eine oder mehrere, bevorzugt alle, Allyl-Einheiten der Ausgangsverbindung peroxidiert werden. Ebenso werden im Schritt (b) mindestens eine oder mehrere, bevorzugt alle, Epoxygruppen geöffnet.

Der Begriff "Polyol" bezeichnet erfindungsgemäß eine Verbindung, die mehr als zwei Hydroxylgruppen aufweist. Der Begriff "Fettsäurepolyol" bezeichnet daher eine Fettsäure, die mehr als zwei Hydroxylgruppen aufweist. Analog bezeichnet der Begriff "Fettsäureesterpolyol" einen Fettsäureester, der mehr als zwei Hydroxylgruppen aufweist.

Erfindungsgemäß ist das "Fettsäurederivat" ausgewählt aus Fettsäureester, Fettether, Fettalkohole, Fettsäureamide, Fettsäurechloride, "Fettsäureamine" bzw. "Fettamine" (im Stand der Technik verwendete Begriffe für Alkylamine, welche aus Fettsäuren synthetisiert wurden) und Fettsäureanhydride. Vorzugsweise ist das Fettsäurederivat ein Fettsäureester.

Soweit nicht anderweitig definiert, werden die Begriffe "ungesättigtes Fettsäurederivat" und "Fettsäurederivat, das mindestens einen ungesättigten Fettsäurerest mit einer oder mehreren Doppelbindungen aufweist" synonym füreinander verwendet.

Ist das Fettsäurederivat ein Fettsäureester, so weist der Fettsäureester mindestens einen ungesättigten Fettsäurerest mit einer oder mehreren Doppelbindungen auf. Daher kann der Fettsäureester sowohl ein Einfachester aus einem Alkohol mit einer ungesättigten Fettsäure, als auch ein Mehrfachester, wie beispielsweise ein Diester oder ein Triester, aus einem mehrwertigen Alkohol, wie z.B. Glycerin, mit mehreren Fettsäuren, von denen mindestens eine ungesättigt ist, sein.

Ist der Fettsäureester ein Mehrfachester, so können die Fettsäurereste des Fettsäureesters gesättigt oder ungesättigt sein, solange mindestens einer der Fettsäurereste des Fettsäureesters mindestens einfach ungesättigt ist. Insbesondere ist es vorteilhaft, wenn mindestens zwei der Fettsäurereste des Fettsäureesters ungesättigt sind. Am meisten bevorzugt ist es, wenn alle Fettsäurereste des Fettsäureesters ungesättigt sind.

Als Alkoholrest des Fettsäureesters können alle im Stand der Technik bekannten Alkoholreste verwendet werden. Beispielsweise ist der Alkoholrest eine Alkylkette mit einer Kettenlänge von 1 bis 6 Kohlenstoffatomen, bevorzugt von 1 bis 4 Kohlenstoffatomen, mehr bevorzugt von 2 bis 4 Kohlenstoffatomen, und am meisten bevorzugt von 3 Kohlenstoffatomen.

Alkohole, die Mono- und/oder Mehrfachester mit Fettsäuren bilden können sind beispielsweise Methanol, Ethanol, 1-Propanol, 2-Propanol, n-Butanol, i-Butanol, tert-Butanol, Glykol, Glycerin, 1,1,1-Trimethylolpropan und Pentaerythrit.

Vorzugsweise handelt es sich bei dem Fettsäureester um ein Glycerid, wie beispielsweise ein Mono-, Di- oder Triglycerid. Besonders bevorzugt handelt es sich bei dem Fettsäureester um ein Triglycerid. Unter den Begriffen "Diglycerid" bzw. "Triglycerid" wird ein zweifach bzw. dreifach mit Fettsäuren verestertes Glycerin verstanden. Dabei können die zwei bzw. drei Fettsäurereste im Di- bzw. Triglycerid gleich oder unterschiedlich sein. Bei dem Diglycerid kann es sich sowohl um ein 1,2-Diglycerid als auch um ein 1,3-Diglycerid handeln.

Bei der ungesättigten Fettsäure oder dem Fettsäurederivat kann es sich um eine natürliche oder synthetisch hergestellte ungesättigten Fettsäure oder ein natürliches oder synthetisch hergestelltes Fettsäurederivat handeln. Vorzugsweise handelt es sich zur Kostenersparnis um eine natürliche ungesättigte Fettsäure oder ein natürliches Fettsäurederivat, wie z.B. einen natürlichen Fettsäureester.

Als Fettsäure oder Fettsäurereste des Fettsäurederivats können alle im Stand der Technik bekannten Fettsäuren oder Fettsäurereste verwendet werden, solange die Fettsäure oder mindestens einer der Fettsäurereste des Fettsäurederivats mindestens eine Doppelbindung aufweist. Beispielsweise weist die Fettsäure oder weisen die Fettsäurereste eine Kettenlänge von 6 bis 30 Kohlenstoffatomen auf, bevorzugt von 10 bis 24 Kohlenstoffatomen, mehr bevorzugt von 14 bis 20 Kohlenstoffatomen, und am meisten bevorzugt von 18 Kohlenstoffatomen.

Die Fettsäure oder das Fettsäurederivat kann substituiert oder unsubstituiert sein. Die möglichen Substituenten der Fettsäure oder des Fettsäurederivats unterliegen keiner besonderen Beschränkung, d.h. anstelle von Wasserstoffatomen können sämtliche im Stand der Technik bekannten Substituenten an den weiteren Positionen der Fettsäure oder des Fettsäurederivats gebunden sein. Beispielsweise sind die möglichen Substituenten ausgewählt aus der Gruppe, bestehend aus Halogen, -NR¹R², -NO₂, -CN, -OR³, -C(O)R⁴, -C(O)NR⁵R⁶, C₁-C₆ Alkyl, C₂-C₆ Alkenyl, C₂-C₆ Epoxygruppe, Aryl, Heteroaryl und -COOR⁷, wobei jedes von R¹ bis R⁷ unabhängig voneinander ein Wasserstoffatom oder eine C₁-C₆ Alkylgruppe sein kann. Die Fettsäure oder das Fettsäurederivat kann eine oder mehrere Epoxygruppen in der Hauptkette enthalten. Vorzugsweise sind die Fettsäurereste unsubstituierte Fettsäurereste, d.h. natürlich vorkommende Fettsäurereste, wie sie beispielsweise in Pflanzenölen vorkommen.

Hier bezeichnet der Begriff "Halogen" insbesondere Fluoratome, Chloratome und Bromatome.

Ferner bezeichnet der Begriff "C₁-C₆ Alkyl" eine verzweigte oder unverzweigte Alkylgruppe mit 1 bis 6 Kohlenstoffatomen, wobei die Alkylgruppe gegebenenfalls wie vorstehend definiert substituiert sein kann.

Der Begriff "C₂-C₆ Alkenyl" bezeichnet eine verzweigte oder unverzweigte Alkenylgruppe mit 2 bis 6 Kohlenstoffatomen, wobei die Alkenylgruppe gegebenenfalls wie vorstehend definiert substituiert sein kann.

Der Begriff "C₂-C₆ Epoxygruppe" bezeichnet eine verzweigte oder unverzweigte Alkyl- oder Alkenylgruppe mit 2 bis 6 Kohlenstoffatomen, die mindestens eine Epoxygruppe enthält und gegebenenfalls wie vorstehend definiert substituiert sein kann.

Der Begriff "Aryl" bezeichnet eine Arylgruppe, bestehend aus beispielsweise 1 bis 3 Ringen, wie beispielsweise eine Phenylgruppe oder eine Naphthylgruppe, wobei die Arylgruppe wie vorstehend definiert substituiert sein kann.

Der Begriff "Heteroaryl" bezeichnet eine Heteroarylgruppe, bestehend aus beispielsweise 1 bis 3 Ringen, wie beispielsweise eine Pyridylgruppe, eine Pyrimidinylgruppe, eine Thienylgruppe, eine Furylgruppe oder eine Pyrrolylgruppe, wobei die Heteroarylgruppe wie vorstehend definiert substituiert sein kann.

Die ungesättigte Fettsäure oder der mindestens eine ungesättigte Fettsäurerest des Fettsäurederivats kann einfach oder mehrfach ungesättigt sein. Die mehrfach ungesättigte Fettsäure oder der mindestens eine mehrfach ungesättigte Fettsäurerest des Fettsäurederivats kann konjugierte oder nicht-konjugierte Doppelbindungen aufweisen. Vorzugsweise weist die mehrfach ungesättigte Fettsäure oder der mindestens eine mehrfach ungesättigte Fettsäurerest des Fettsäurederivats nicht-konjugierte Doppelbindungen auf.

Vorzugsweise ist die Fettsäure oder sind die Fettsäurereste des Fettsäurederivats einfach ungesättigt. Insbesondere ist es vorteilhaft, wenn die Fettsäure Ölsäure ist oder wenn mindestens ein Fettsäurerest des Fettsäurederivats ein Ölsäurerest ist. Noch vorteilhafter ist es, wenn alle Fettsäurereste des Fettsäurederivats Ölsäurereste sind, insbesondere wenn das Fettsäurederivat ein Fettsäureester ist.

Ist das Fettsäurederivat ein Fettsäureester, so stammt der Fettsäureester vorzugsweise von high-oleic Ölen. High-oleic Öle sind Triglyceride mit einem sehr hohen Ölsäureanteil. Vorzugsweise stammt der Fettsäureester von high-oleic Ölen mit einem Ölsäureanteil von mindestens 60%, noch bevorzugter von mindestens 70%, noch bevorzugter von mindestens 80%, noch bevorzugter von mindestens 90% und am bevorzugtesten von 100%, basierend auf der Gesamtzahl der Fettsäurereste in den Triglyceriden.

Im Schritt (a) des erfindungsgemäßen Verfahrens wird eine Allyl-Einheit (siehe allgemeine Formel (1), bei welcher optional vorhandene Substituenten nicht dargestellt sind) (bzw. alle Allyl-Einheiten) der ungesättigten Fettsäure oder des ungesättigten Fettsäurederivats peroxidert.

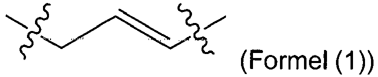

Es bildet sich dabei eine Allylhydroperoxid-Einheit (siehe allgemeine Formel (2), bei welcher optional vorhandene Substituenten ebenfalls nicht dargestellt sind), die anschließend zu einer Epoxyalkohol-Einheit umgesetzt wird.

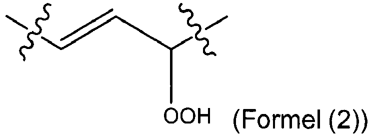

Erfindungsgemäß wird die Allylhydroperoxid-Einheit zu einer Epoxyalkohol-Einheit (siehe allgemeine Formel (3), bei welcher optional vorhandene Substituenten ebenfalls nicht dargestellt sind) umgesetzt.

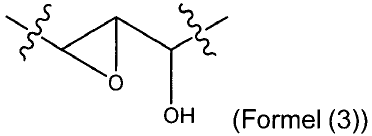

Der Begriff "Epoxyalkohol" bezeichnet erfindungsgemäß eine Verbindung, die eine Epoxyalkohol-Einheit der allgemeinen Formel (3) umfasst.

Bei nicht-konjugierten, nicht-endständigen Doppelbindungen kann es zwei mögliche Allyl-Einheiten geben, an denen die Peroxidation erfolgen kann. Beispielsweise besitzt ein Ölsäurerest zwei CH₂-Gruppen, die an die Kohlenstoffatome der Doppelbindung (C9-C10) gebunden sind. Daher kann die Peroxidation an zwei möglichen Allyl-Einheiten (C8 bis C10 oder C9 bis C11) erfolgen, wodurch zwei unterschiedliche Regioisomere (8,9,10- und 9,10,11-Triolderivate) als Polyol erhalten werden können.

Durch das erfindungsgemäße Verfahren kann, je nach Durchführung, beispielsweise aus Trioleat Trihydroxystearinsäure (das 8,9,10- und das 9,10,11-Regioisomer) oder ein entsprechendes Triglyceridpolyol synthetisiert werden.

Das Reaktionsmedium (Medium) im Schritt (a) des erfindungsgemäßen Verfahrens unterliegt keinen besonderen Beschränkungen. Beispielsweise kann es sich bei dem Medium um ein wässriges Medium, ein Medium auf Basis eines oder mehrerer organischer Lösungsmittel oder um ein Medium auf Basis eines Gemisches aus Wasser und einem oder mehreren organischen Lösungsmitteln handeln. Vorzugsweise wird das Reaktionsmedium für Schritt (a) des erfindungsgemäßen Verfahrens ausgewählt aus der Gruppe, bestehend aus Dichlormethan, Chloroform, Hexan und Cyclohexan. Am bevorzugtesten ist das Reaktionsmedium für Schritt (a) des erfindungsgemäßen Verfahrens Dichlormethan, da dieses zu den besten Ausbeuten führt.

Die Konzentration der ungesättigten Fettsäure oder des Fettsäurederivats in dem Reaktionsmedium unterliegt keinen besonderen Einschränkungen. Beispielsweise kann die ungesättigte Fettsäure oder das Fettsäurederivat in einer Konzentration von 0,5 bis 4 M, insbesondere von 0,7 bis 3 M, von 0,9 bis 2,5 M, von 1,2 bis 2,2 M, und am meisten bevorzugt von 1,5 bis 2,0 M vorliegen. Bei Konzentrationen von über 4 M können verlängerte Reaktionszeiten nötig sein. Bei Konzentrationen von unter 0,5 M müssen große Mengen an Lösungsmittel verwendet werden.

Erfindungsgemäß erfolgt Schritt (a) in Gegenwart eines Katalysators, d.h. katalytisch.

Im erfindungsgemäßen Verfahren erfolgt Schritt (a) in Gegenwart eines Übergangsmetallkatalysators, wie beispielsweise Titanisopropoxid (Ti(OiPr)₄), Vanadiumoxyacetylacetonat oder Molybdänoxyacetylacetonat. Der Übergangsmetallkatalysator katalysiert die Umsetzung der Allylhydroperoxid-Einheit zur Epoxyalkohol-Einheit. Vorzugsweise ist der Übergangsmetallkatalysator Titanisopropoxid (Ti(OiPr)₄). Bei Verwendung von Titanisopropoxid (Ti(OiPr)₄) als Übergangsmetallkatalysator kommt es aufgrund dessen geringer Lewis-Acidität und dem fehlenden Redoxverhalten zu keinen unerwünschten Nebenreaktionen.

Beispielsweise kann der Übergangsmetallkatalysator in einer Konzentration von 1 bis 10 mol%, insbesondere von 2 bis 8 mol%, von 3 bis 7 mol%, von 4 bis 6 mol%, und am meisten bevorzugt von 4,5 bis 5,0 mol%, bezogen auf die Konzentration der ungesättigten Fettsäure (pro Doppelbindung) oder des Fettsäurederivats (pro Doppelbindung), vorliegen. Abhängig vom Substrat kann der Übergangsmetallkatalysator nicht nur in obigen katalytischen Mengen, sondern auch in stöchiometrischen Mengen oder in kleineren als den angegeben Mengen vorhanden sein.

Erfindungsgemäß handelt es sich bei der Katalyse im Schritt (a) um eine in situ Katalyse.

Erfindungsgemäß erfolgt Schritt (a) in Gegenwart eines Peroxidationsmittels, insbesondere von Sauerstoff. Dabei kann beispielsweise Umgebungsluft verwendet werden und/oder Sauerstoff in das Medium geleitet werden. Bevorzugt wird Sauerstoff in das Medium geleitet. Erfindungsgemäß wird Singulettsauerstoff als Peroxidationsmittel verwendet, wobei der Singulettsauerstoff sowohl chemisch als auch photochemisch gebildet werden kann.

Erfindungsgemäß erfolgt die Peroxidation in Schritt (a) durch eine Photoperoxidation, d.h. durch Licht stimuliert. Die Lichtquelle unterliegt keinen besonderen Beschränkungen. Beispielswiese kann eine Natriumdampflampe mit einer Emissionswellenlänge bei 589 nm verwendet werden. Das Licht, das von der Lichtquelle emittiert wird, wird dazu verwendet, einen Photosensibilisator anzuregen, der in dem Wellenlängenbereich des Lichtes, das von der Lichtquelle emittiert wird, einen Absorptionsbereich aufweist.

Daher erfolgt im erfindungsgemäßen Verfahren Schritt (a) in Gegenwart eines Photosensibilisators, wie beispielsweise Tetraphenylporphyrin (TPP), der Energie auf einen anderen Stoff übertragen kann. Beispielsweise kann der angeregte Photosensibilisator Energie auf Sauerstoff übertragen, um Singulettsauerstoff zu generieren. Zum Beispiel kann Tetraphenylporphyrin (TPP), nach Anregung durch Licht einer Natriumdampflampe, zur Erzeugung von Singulettsauerstoff verwendet werden. Der Photosensibilisator kann beispielsweise in einer Konzentration von 0,005 bis 0,020 mol%, insbesondere von 0,007 bis 0,017 mol%, mehr bevorzugt von 0,005 bis 0,015 mol%, bezogen auf die Konzentration der ungesättigten Fettsäure (pro Doppelbindung) oder des Fettsäurederivats (pro Doppelbindung), vorliegen.

In einer bevorzugten Ausführungsform des erfindungsgemäßen Verfahrens erfolgt Schritt (a) in einem Temperaturbereich von 20°C bis 35°C, vorzugsweise in einem Temperaturbereich von 20°C bis 30°C, noch bevorzugter von 21°C bis 27°C, noch bevorzugter von 23°C bis 27°C und am bevorzugtesten von 25°C bis 27°C.

Die Reaktionszeit im Schritt (a) unterliegt keinen besonderen Beschränkungen. Sie kann beispielsweise in einem Bereich von 1 bis 20 h, vorzugsweise in einem Bereich von 5 bis 18 h, noch bevorzugter von 9 bis 16 h, noch bevorzugter von 9 bis 13 h und am bevorzugtesten von 12 bis 13 h liegen.

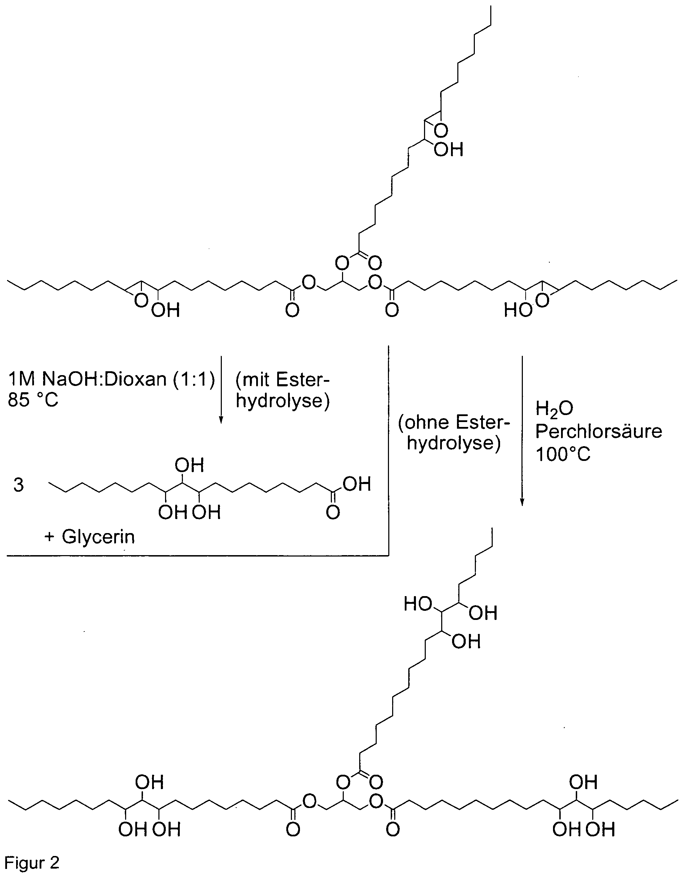

Im Schritt (b) des erfindungsgemäßen Verfahrens erfolgt das Öffnen der Epoxygruppe (bzw. aller Epoxygruppen) der entstandenen Epoxyalkohol-Einheit durch Hydrolyse, wobei ein Polyol gebildet wird (siehe beispielsweise Figur 2). Aus der Epoxyalkohol-Einheit der Formel (3) bildet sich dabei eine Triol-Einheit (siehe allgemeine Formel (4), bei welcher optional vorhandene Substituenten ebenfalls nicht dargestellt sind).

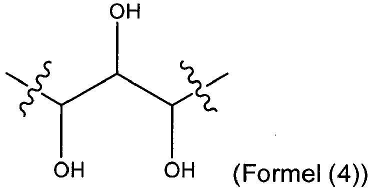

Das Reaktionsmedium im Schritt (b) des erfindungsgemäßen Verfahrens ist ein wässriges Lösungsmittel. Beispielsweise kann es sich um Wasser oder um ein Gemisch aus Wasser und einem oder mehreren organischen Lösungsmitteln handeln. Die einen oder mehreren organischen Lösungsmittel sind vorzugsweise mit Wasser mischbar und vorzugsweise darauf ausgelegt den Epoxyalkohol zu lösen. Bei einem Wasser/Lösungsmittel-Gemisch unterliegt das Mischverhältnis von Wasser mit dem mindestens einen organischen Lösungsmittel keinen besonderen Beschränkungen. Beispielsweise kann ein Volumenmischverhältnis von Wasser mit dem mindestens einen organischen Lösungsmittel von 99:1 bis 1:99, insbesondere von 10:90 bis 90:10, von 30:70 bis 70:30, von 40:60 bis 60:40, und am meisten bevorzugt von 50:50 vorliegen. Werden mehrere organische Lösungsmittel mit Wasser gemischt, so unterliegt deren Mischverhältnis keinen besonderen Beschränkungen. Beispielsweise können sie zu gleichen Volumenteilen vorliegen. Vorzugsweise wird das Reaktionsmedium für Schritt (b) des erfindungsgemäßen Verfahrens ausgewählt aus der Gruppe, bestehend aus Wasser, wässrigem Dioxan, wässrigem Methanol, wässrigem Ethanol, wässrigem 2-Propanol, wässrigem Aceton, wässrigem Acetonitril, wässrigem Dimethylsulfoxid (DMSO), wässrigem Dimethylformamid (DMF), wässrigem Glycerin, wässrigem Ethylenglykol und wässrigem Tetrahydrofuran (THF).

Ist das Fettsäurederivat ein Fettsäureester, so lassen sich je nach pH-Wert des Mediums unterschiedliche Polyole aus dem Epoxyalkohol erhalten (vgl. Figur 2). Dies lässt sich darauf zurückführen, dass bei pH ≤ 7 die Esterbindungen des Fettsäureesters nach der Hydrolyse des Epoxyalkohols erhalten bleiben, wodurch ein Fettsäureesterpolyol, beispielsweise ein Triglyceridpolyol, entsteht. Bei pH ≤ 7 ist das mindestens eine gebildete Polyol somit hauptsächlich ein Fettsäurepolyol (beim Edukt Fettsäure) oder ein Fettsäureesterpolyol (beim Edukt Fettsäureester). Bei einem pH > 7 kann neben der Hydrolyse des Epoxyalkohols die teilweise oder vollständige Hydrolyse der Esterbindungen des Fettsäureesters erfolgen, wodurch beispielsweise bei einem Triglycerid als Fettsäureester Glycerin und 3 entsprechende Fettsäuren (bzw. deren Salze), aber auch beispielsweise entsprechende Mono- und Diglyceride neben der jeweiligen Anzahl an entsprechenden (abgespaltenen) Fettsäuren (bzw. deren Salze), gebildet werden können. Bei pH > 7 ist das mindestens eine gebildete Polyol daher ein Fettsäurepolyol (bzw. dessen Salz) und/oder (meist in geringeren Mengen) ein Fettsäureesterpolyol, das aus dem ursprünglichen Fettsäureester durch Abspaltung von keinem oder mindestens einem Fettsäurerest erhalten wird.

Somit erfolgt in einer Ausführungsform des erfindungsgemäßen Verfahrens Schritt (b) bei pH > 7, wobei das mindesteins eine gebildete Polyol bei vollständiger Hydrolyse der Esterbindungen ein Fettsäurepolyol ist, das pro Doppelbindung der ursprünglichen ungesättigten Fettsäure oder pro Doppelbindung des entsprechenden Fettsäurerests des ursprünglichen Fettsäureesters mehr als 2 Hydroxylgruppen aufweist. Die Zahl an Hydroxylgruppen des gebildeten Fettsäurepolyols pro Doppelbindung der ursprünglichen ungesättigten Fettsäure oder pro Doppelbindung des entsprechenden Fettsäurerests des ursprünglichen Fettsäureesters ergibt sich dabei aus dem Mittel aller ungesättigten Fettsäuremoleküle und/oder aller entsprechenden ungesättigten Fettsäurereste aller Fettsäureestermoleküle. Theoretisch können pro Doppelbindung der ursprünglichen ungesättigten Fettsäure oder pro Doppelbindung eines Fettsäurerestes des ursprünglichen Fettsäureesters bis zu 3 Hydroxylgruppen erhalten werden. Diese Zahl kann sich beispielsweise dadurch verringern, dass, bezogen auf alle ungesättigten Fettsäuremoleküle und/oder alle entsprechenden ungesättigten Fettsäurereste aller Fettsäureestermoleküle, ein Teil der Doppelbindungen gar nicht umgesetzt wird, ein Teil der Epoxyalkohol-Einheiten nicht geöffnet wird und/oder ein Teil der Allylhydroperoxid-Einheiten in eine entsprechende Allylhydroxid-Einheit umgesetzt wird. Wurden beispielsweise 75% der gesamten Epoxyalkohol-Einheiten (Formel (3)) aller ungesättigten Fettsäuremoleküle und/oder aller entsprechenden ungesättigten Fettsäurereste aller Fettsäureestermoleküle zu entsprechenden Triol-Einheiten (Formel (4)) umgesetzt, während die restlichen 25% der gesamten Epoxyalkohol-Einheiten nicht weiter umgesetzt wurden, so ergibt sich ein Wert von 2,5. Ebenso ergibt sich ein Wert von 2,5 beispielsweise wenn 83,33% der gesamten Doppelbindungen (bzw. Allyl-Einheiten) aller ungesättigten Fettsäuremoleküle und/oder aller entsprechenden ungesättigten Fettsäurereste aller Fettsäureestermoleküle zu entsprechenden Triol-Einheiten (Formel (4)) umgesetzt wurden, während die restlichen 16,67% der gesamten Doppelbindungen (bzw. Allyl-Einheiten) nicht umgesetzt wurden. Insbesondere ist das mindestens eine gebildete Polyol ein Fettsäurepolyol, das pro Doppelbindung der ursprünglichen ungesättigten Fettsäure oder pro Doppelbindung des entsprechenden Fettsäurerests des ursprünglichen Fettsäureesters mindestens 2,3 Hydroxylgruppen, mindestens 2,5 Hydroxylgruppen, mindestens 2,7 Hydroxylgruppen, noch bevorzugter mindestens 2,9 Hydroxylgruppen aufweist. Am meisten bevorzugt ist das mindestens eine gebildete Polyol ein Fettsäurepolyol, das pro Doppelbindung der ursprünglichen ungesättigten Fettsäure oder pro Doppelbindung des entsprechenden Fettsäurerests des ursprünglichen Fettsäureesters 3 Hydroxylgruppen aufweist. Die vorstehende Definition ist auf ein Fettsäureesterpolyol, das aus dem ursprünglichen Fettsäureester durch Abspaltung von keinem oder mindestens einem Fettsäurerest erhalten wird, und die möglicherweise abgespaltenen Fettsäuren analog anwendbar.

Vorzugsweise wird das Medium, das bei pH > 7 verwendet wird, ausgewählt aus der Gruppe bestehend aus wässrigem Dioxan, wässrigem Methanol, wässrigem Ethanol, wässrigem 2-Propanol, wässrigem Aceton, wässrigem Acetonitril, wässrigem Dimethylsulfoxid (DMSO), wässrigem Dimethylformamid (DMF), wässrigem Glycerin, wässrigem Ethylenglykol und wässrigem Tetrahydrofuran (THF). Besonders bevorzugt ist das Medium, das bei pH > 7 verwendet wird, wässriges Dioxan.

Die Konzentration der Fettsäure, welche die Epoxyalkohol-Einheit umfasst, oder des Fettsäureesters, der die Epoxyalkohol-Einheit umfasst, in dem Reaktionsmedium, das bei pH > 7 verwendet wird, unterliegt keinen besonderen Einschränkungen. Beispielsweise kann die Fettsäure, welche die Epoxyalkohol-Einheit umfasst, oder der Fettsäureester, der die Epoxyalkohol-Einheit umfasst, in einer Konzentration von 0,01 bis 2,0 M, insbesondere von 0,1 bis 1,0 M, von 0,15 bis 0,75 M, von 0,2 bis 0,5 M, und am meisten bevorzugt von 0,3 bis 0,4 M vorliegen.

Vorzugsweise erfolgt die Hydrolyse bei pH > 7 in Gegenwart einer Base, wie beispielsweise Natronlauge (NaOH), Kalilauge (KOH), Ammoniak (NH₃), Calciumhydroxid (Ca(OH)₂), Natriumcarbonat (Na₂CO₃) und Kaliumcarbonat (K₂CO₃). Beispielsweise kann die Base in einer Konzentration von 0,05 bis 3 M, insbesondere von 0,1 bis 2 M, von 0,2 bis 1,0 M, von 0,3 bis 0,7 M, und am meisten bevorzugt von 0,4 bis 0,6 M vorliegen.

Die Temperatur im Schritt (b) bei pH > 7 unterliegt keinen besonderen Beschränkungen. Vorzugsweise erfolgt die Hydrolyse bei pH > 7 in einem Temperaturbereich von 20°C bis 100°C, vorzugsweise in einem Temperaturbereich von 60°C bis 100°C, noch bevorzugter von 70°C bis 95°C, noch bevorzugter von 80°C bis 90°C und am meisten bevorzugt bei 85°C.

Die Reaktionszeit im Schritt (b) bei pH > 7 unterliegt keinen besonderen Beschränkungen. Sie kann beispielsweise in einem Bereich von 10 bis 25 h, vorzugsweise in einem Bereich von 14 bis 24 h, noch bevorzugter von 15 bis 22 h, noch bevorzugter von 16 bis 21 h und am meisten bevorzugt von 18 bis 20 h liegen. Im Durchschnitt kann eine Reaktionszeit von 48 h als ausreichend angesehen werden.

Wie vorstehend definiert, ist die ungesättigte Fettsäure vorzugsweise Ölsäure oder umfasst der Fettsäureester vorzugsweise mindestens einen Ölsäurerest. Daher ist das mindestens eine Polyol in dieser bevorzugten Ausführungsform durch die Hydrolyse bei pH > 7 mindestens ein Fettsäurepolyol, ausgewählt aus 8,9,10- und 9,10,11-Trihydroxystearinsäure (bzw. deren Salze), und/oder mindestens ein Fettsäureesterpolyol mit mindestens einem 8,9,10- oder 9,10,11-Trihydroxystearinsäurerest, wobei das Molverhältnis der Regioisomere beispielsweise 50:50, basierend auf der Gesamtheit der Regioisomere, sein kann.

In einer weiteren Ausführungsform des erfindungsgemäßen Verfahrens erfolgt Schritt (b) bei pH ≤ 7, wobei das mindestens eine gebildete Polyol ein Fettsäurepolyol oder ein Fettsäureesterpolyol ist und das gebildete Polyol pro Doppelbindung der ursprünglichen ungesättigten Fettsäure oder pro Doppelbindung des ursprünglichen Fettsäureesters mehr als 2 Hydroxylgruppen aufweist. Die Zahl an Hydroxylgruppen des mindestens einen gebildeten Polyols pro Doppelbindung der ursprünglichen ungesättigten Fettsäure oder pro Doppelbindung des ursprünglichen Fettsäureesters ergibt sich dabei aus dem Mittel aller ungesättigten Fettsäuremoleküle und/oder aller ungesättigten Fettsäurereste aller Fettsäureestermoleküle. Theoretisch können pro Doppelbindung der ursprünglichen ungesättigten Fettsäure oder pro Doppelbindung eines Fettsäurerestes des ursprünglichen Fettsäureesters bis zu 3 Hydroxylgruppen erhalten werden. Diese Zahl kann sich beispielsweise dadurch verringern, dass, bezogen auf alle ungesättigten Fettsäuremoleküle und/oder alle ungesättigten Fettsäurereste aller Fettsäureestermoleküle, ein Teil der Doppelbindungen gar nicht umgesetzt wird, ein Teil der Epoxyalkohol-Einheiten nicht geöffnet wird und/oder ein Teil der Allylhydroperoxid-Einheit in eine entsprechende Allylhydroxid-Einheit umgesetzt wird. Wurden beispielsweise 75% der gesamten Epoxyalkohol-Einheiten (Formel (3)) aller ungesättigten Fettsäuremoleküle und/oder aller ungesättigten Fettsäurereste aller Fettsäureestermoleküle zu entsprechenden Triol-Einheiten (Formel (4)) umgesetzt, während die restlichen 25% der gesamten Epoxyalkohol-Einheiten nicht weiter umgesetzt wurden, so ergibt sich ein Wert von 2,5. Ebenso ergibt sich ein Wert von 2,5 beispielsweise wenn 83,33% der gesamten Doppelbindungen (bzw. Allyl-Einheiten) aller ungesättigten Fettsäuremoleküle und/oder aller ungesättigten Fettsäurereste aller Fettsäureestermoleküle zu entsprechenden Triol-Einheiten (Formel (4)) umgesetzt wurden, während die restlichen 16,67% der gesamten Doppelbindungen (bzw. Allyl-Einheiten) nicht umgesetzt wurden. Insbesondere ist das mindestens eine gebildete Polyol ein Polyol (Fettsäurepolyol oder Fettsäureesterpolyol, wie beispielsweise ein Triglyceridpolyol), das pro Doppelbindung der ursprünglichen ungesättigten Fettsäure oder pro Doppelbindung des ursprünglichen Fettsäureesters mindestens 2,3 Hydroxylgruppen, mindestens 2,5 Hydroxylgruppen, mindestens 2,7 Hydroxylgruppen, noch bevorzugter mindestens 2,9 Hydroxylgruppen aufweist. Am meisten bevorzugt ist das mindestens eine gebildete Polyol ein Polyol (Fettsäurepolyol oder Fettsäureesterpolyol, wie beispielsweise ein Triglyceridpolyol), das pro Doppelbindung der ursprünglichen ungesättigten Fettsäure oder pro Doppelbindung des ursprünglichen Fettsäureesters 3 Hydroxylgruppen aufweist.

Vorzugsweise ist das Medium, das bei pH ≤ 7 verwendet wird, Wasser.

Die Konzentration der Fettsäure, welche die Epoxyalkohol-Einheit umfasst, oder des Fettsäureesters, der die Epoxyalkohol-Einheit umfasst, in dem Reaktionsmedium, das bei pH ≤ 7 verwendet wird, unterliegt keinen besonderen Einschränkungen. Beispielsweise kann die Fettsäure, welche die Epoxyalkohol-Einheit umfasst, oder der Fettsäureester, der die Epoxyalkohol-Einheit umfasst, in einer Konzentration von 0,001 bis 3 M, insbesondere von 0,01 bis 2 M, von 0,03 bis 1 M, von 0,05 bis 0,5 M, von 0,07 bis 0,3 M, und am meisten bevorzugt von 0,08 bis 0,2 M vorliegen.

Vorzugsweise erfolgt die Hydrolyse bei pH ≤ 7 in Gegenwart von Perchlorsäure (HClO₄). Beispielsweise kann Perchlorsäure (HClO₄) in einer Konzentration von 0,01 bis 5 M, insbesondere von 0,1 bis 4 M, von 0,3 bis 3 M, von 0,5 bis 2 M, von 0,7 bis 1,5 M, und am meisten bevorzugt von 0,8 bis 1,2 M vorliegen.

Vorzugsweise erfolgt die Hydrolyse bei pH ≤ 7 in Gegenwart einer Mischung aus Wasser und Perchlorsäure.

Die Temperatur im Schritt (b) bei pH ≤ 7 unterliegt keinen besonderen Beschränkungen. Vorzugsweise erfolgt die Hydrolyse bei pH ≤ 7 in einem Temperaturbereich von 20°C bis 100°C, vorzugsweise in einem Temperaturbereich von 40°C bis 100°C, noch bevorzugter von 60°C bis 100°C, noch bevorzugter von 80°C bis 100°C, noch bevorzugter von 85°C bis 100°C und am meisten bevorzugt von 90°C bis 100°C.

Die Reaktionszeit im Schritt (b) bei pH ≤ 7 unterliegt keinen besonderen Beschränkungen. Sie kann beispielsweise in einem Bereich von 0,1 bis 75 h, vorzugsweise in einem Bereich von 1 bis 75 h, noch bevorzugter von 10 bis 75 h, noch bevorzugter von 20 bis 75 h, noch bevorzugter von 40 bis 75 h, noch bevorzugter von 50 bis 75 h, noch bevorzugter von 60 bis 75 h, noch bevorzugter von 70 bis 75 h und am meisten bevorzugt von 70 bis 72 h liegen. Im Durchschnitt kann eine Reaktionszeit von 48 h als ausreichend angesehen werden.

Wie vorstehend definiert, ist die ungesättigte Fettsäure vorzugsweise Ölsäure oder umfasst der Fettsäureester vorzugsweise mindestens einen Ölsäurerest. Daher ist in dieser bevorzugten Ausführungsform durch die Hydrolyse bei pH ≤ 7 das mindestens eine gebildete Polyol mindestens eines, ausgewählt aus 8,9,10- und 9,10,11-Trihydroxystearinsäure (bzw. deren Salze), und/oder mindestens ein Fettsäurerest des gebildeten Polyols ein 8,9,10- oder 9,10,11-Trihydroxystearinsäurerest. Dabei kann das Molverhältnis der Regioisomere beispielsweise 50:50, basierend auf der Gesamtheit der Regioisomere, sein.

Ist das Fettsäurederivat kein Fettsäureester, so lassen sich je nach pH-Wert des Mediums gegebenenfalls ebenfalls unterschiedliche Polyole aus dem Epoxyalkohol erhalten. Die vorstehenden Definitionen und bevorzugten Ausführungsformen bezüglich der pro Doppelbindung (des entsprechenden Fettsäurerests) des ursprünglichen Fettsäureesters entstehenden Hydroxylgruppen, des verwendeten Mediums, der Konzentration des Fettsäureesters in dem Reaktionsmedium, der verwendeten Säure oder Base, des Temperaturbereichs, der Reaktionszeit und des mindestens einen gebildeten Polyols und/oder der Fettsäurereste des mindestens einen gebildeten Polyols wird der Fachmann analog auf andere Derivate anwenden bzw. anpassen.

Das erfindungsgemäße Verfahren kann als Ausgangsmaterial sowohl eine reine ungesättigte Fettsäure als auch ein Gemisch aus zwei oder mehr ungesättigten Fettsäuren oder sowohl ein reines Fettsäurederivat als auch ein Gemisch aus zwei oder mehr verschiedenen Fettsäurederivaten verwenden. Ebenso kann ein Gemisch aus mindestens einer ungesättigten Fettsäure und mindestens einem Fettsäurederivat als Ausgangsmaterial verwendet werden. Insbesondere kann die ungesättigte Fettsäure in Schritt (a) alleine oder im Gemisch mit einer oder mehreren unterschiedlichen gesättigten oder ungesättigten Fettsäuren vorliegen, oder das Fettsäurederivat, das mindestens einen ungesättigten Fettsäurerest mit einer oder mehreren Doppelbindungen aufweist, in Schritt (a) alleine oder im Gemisch mit einem oder mehreren unterschiedlichen gesättigten oder ungesättigten Fettsäurederivaten vorliegen oder im Schritt (a) ein Gemisch von einem oder mehreren unterschiedlichen Fettsäuren und einem oder mehreren unterschiedlichen Fettsäurederivaten vorliegen, wobei mindestens eine Fettsäure oder ein Fettsäurederivat ungesättigt ist.

Die vorliegende Erfindung ermöglicht eine neuartige einfache Zweistufensynthese von Polyolen, wobei es vorteilhafterweise möglich ist, aus beispielsweise einem mindestens einfach ungesättigten Triglycerid je nach Durchführung entweder ein Triglyceridpolyol oder eine Trihydroxyfettsäure zu bilden. Insbesondere ist es durch das erfindungsgemäße Verfahren vorteilhafterweise möglich, Polyole mit mehr als 2 Hydroxylgruppen pro Doppelbindung der ursprünglichen ungesättigten Fettsäure oder pro Doppelbindung des entsprechenden Fettsäurerests des ursprünglichen Fettsäurederivats zu bilden. Es kann daher beispielsweise von einem Triglycerid mit einer Doppelbindung pro Fettsäurerest als Ausgangsmaterial ausgegangen werden, um ein Polyol mit einer hohen Anzahl von Hydroxylfunktionalitäten zu erhalten. Bisher war eine vergleichbare Anzahl an Hydroxylfunktionalitäten nur bei der Verwendung von Rizinusöl, in dessen Struktur bereits Hydroxylgruppen vorhanden sind, oder dem Einsatz von polyfunktionellen Reagenzien möglich. Im Gegensatz zu anderen Ölen mit hohem Anteil an ungesättigten Fettsäuren, ist Rizinusöl jedoch sehr teuer.

Die Figuren zeigen:
Fig. 1: Schritt (a) des erfindungsgemäßen Verfahrens am Beispiel des Triglycerids Trioleat (nur ein Regioisomer gezeigt).
Fig. 2: Schritt (b) des erfindungsgemäßen Verfahrens am Beispiel des Epoxyalkohols, der im Schritt (a) aus dem Triglycerid Trioleat erhalten wurde (nur ein Regioisomer gezeigt).

Die vorliegende Erfindung wird anhand der folgenden, nicht-einschränkenden Beispiele näher erläutert.

### Allgemeines:

Die Zahl der Doppelbindungen in den verwendeten Ausgangssubstanzen bzw. der Epoxy- und/oder der Hydroxyl-Gruppen in den (Zwischen-)Produkten wurde mittels ¹H-NMR in CDCl₃ bei 300 MHz bestimmt, wobei Mittelwerte erhalten wurden. Die Umsatzkontrolle erfolgte ebenfalls über ¹H-NMR.

### Beispiel 1: Photoperoxidation von Trioleat

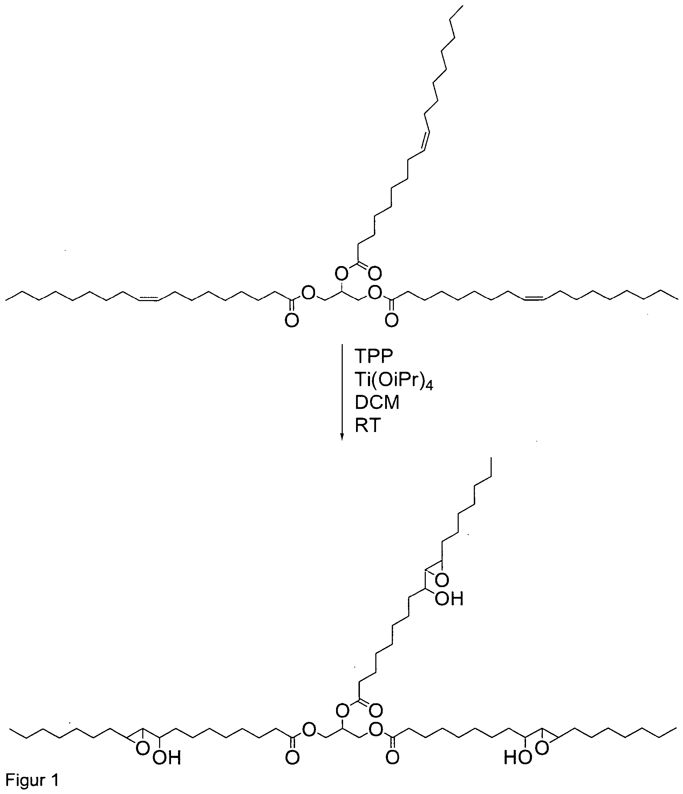

Ein Trioleat-Öl mit 2,9 Doppelbindungen pro Triglycerid (bestimmt mittels ¹H-NMR) wurde mit 0,014 mol% Tetraphenylporphyrin (TPP) und 5 mol% Ti(OiPr)₄ in Dichlormethan (1,2 M) bei Raumtemperatur umgesetzt. Es erfolgte die Bildung der Allylhydroperoxid-Einheiten durch Photoperoxidation mit Sauerstoff und anschließend die Umlagerung der Allylhydroperoxid-Einheiten zu den entsprechenden Epoxyalkohol-Einheiten unter dem Einfluss von Titanisopropoxid (Figur 1). Für die Photoperoxidation wurde dabei ein quantitativer Umsatz der einfach ungesättigten Doppelbindungen in 9 h erreicht (Umsatzkontrolle mittels ¹H-NMR). Anschließend wurde das Lösungsmittel bei vermindertem Druck entfernt. Die entsprechenden Epoxyalkoholtriglyceride wurden dabei als 8,9,10-/9,10,11-Isomerengemisch mit durchschnittlich 2,5 Epoxygruppen und 2,8 Hydroxylgruppen pro Triglycerid erhalten.

### Beispiel 2: Epoxidringöffnung des Produkts aus Beispiel 1 bei pH ≤ 7

Das Produkt (0,1 M) aus Beispiel 1 wurde mit einem Gemisch aus Wasser und 14 Gew.-% 70%-iger Perchlorsäure bei 100°C für 72 h umgesetzt. Danach wurde mit Chloroform extrahiert und mit Wasser gewaschen. Die organische Phase wurde über Natriumsulfat getrocknet und mit Aktivkohle versetzt, um das TPP zu entfernen. Anschließend wurde das Chloroform bei vermindertem Druck entfernt. Es wurde ein entsprechendes Polyol mit 7,5 Hydroxylgruppen erhalten (Figur 2). Es wurde ein Umsatz der Doppelbindungen in Hydroxylgruppen zu 86 %, basierend auf den 2,9 Doppelbindungen pro Triglycerid in dem Ausgangs-Trioleat-Öl, erreicht.

### Beispiel 3: Epoxidrinqöffnunq des Produkts aus Beispiel 1 bei pH ≥ 7

Das Produkt aus Beispiel 1 wurde mit einem Gemisch aus 1 molarer verdünnter Natronlauge und Dioxan (Mischung 1:1) bei 85°C umgesetzt. Danach wurde Dioxan unter vermindertem Druck entfernt und anschließend der Rückstand mit 1 molarer Salzsäure angesäuert. Nach Extraktion mit Ethylacetat wurde zwei Mal mit Wasser gewaschen um Glycerin zu entfernen. Die organische Phase wurde über Natriumsulfat getrocknet und mit Aktivkohle versetzt, um das TPP zu entfernen. Anschließend wurde das Ethylacetat bei vermindertem Druck entfernt. Es wurde Trihydroxystearinsäure als Gemisch der 8,9,10-/9,10,11-Regioisomeren bei einem Isomerenverhältnis von 50:50 erhalten (Figur 2). Dabei wird ein Umsatz der Doppelbindungen in Hydroxylgruppen zu 86 %, basierend auf den 2,9 Doppelbindungen pro Triglycerid in dem Ausgangs-Trioleat-Öl, erreicht.

## Patentansprüche

1. Verfahren zur Herstellung eines Polyols aus einer ungesättigten Fettsäure mit einer oder mehreren Doppelbindungen oder eines Polyols aus einem Fettsäurederivat, ausgewählt aus Fettsäureester, Fettether, Fettalkohole, Fettsäureamide, Fettsäurechloride, Fettamine und Fettsäureanhydride, das mindestens einen ungesättigten Fettsäurerest mit einer oder mehreren Doppelbindungen aufweist, umfassend die Schritte
(a) Peroxidieren einer Allyl-Einheit der ungesättigten Fettsäure oder des ungesättigten Fettsäurederivats unter Bildung einer Allylhydroperoxid-Einheit, wobei anschließend die Allylhydroperoxid-Einheit zu einer Epoxyalkohol-Einheit umgesetzt wird, und
(b) Öffnen der Epoxygruppe der entstandenen Epoxyalkohol-Einheit durch Hydrolyse unter Bildung des Polyols,
wobei Schritt (a) in Gegenwart eines Übergangsmetallkatalysators erfolgt,
die Peroxidation der Allyl-Einheit in Schritt (a) eine Photoperoxidation in Gegenwart eines Peroxidationsmittels und eines Photosensibilisators ist, wobei Singulettsauerstoff als Peroxidationsmittel verwendet wird, und wobei
die Katalyse in Schritt (a) *in situ* erfolgt.

2. Verfahren nach Anspruch 1, wobei der Übergangsmetallkatalysator ein Übergangsmetallkatalysator, ausgewählt aus der Gruppe, bestehend aus Titanisopropoxid, Vanadiumoxyacetylacetonat und Molybdänoxyacetylacetonat, ist.

3. Verfahren nach Anspruch 1 oder 2, wobei das Fettsäurederivat ein Fettsäureester ist.

4. Verfahren nach Anspruch 3, wobei Schritt (b) bei pH > 7 erfolgt und das gebildete Polyol ein Fettsäurepolyol und/oder ein Fettsäureesterpolyol ist und das gebildete Polyol pro Doppelbindung der ursprünglichen ungesättigten Fettsäure oder pro Doppelbindung des entsprechenden Fettsäurerests des ursprünglichen Fettsäureesters mehr als 2 Hydroxylgruppen aufweist.

5. Verfahren nach Anspruch 4, wobei Schritt (b) in Gegenwart einer Base, ausgewählt aus der Gruppe, bestehend aus Natronlauge (NaOH), Kalilauge (KOH), Ammoniak (NH₃), Calciumhydroxid (Ca(OH)₂), Natriumcarbonat (Na₂CO₃) und Kaliumcarbonat (K₂CO₃), erfolgt.

6. Verfahren nach Anspruch 4 oder 5, wobei das gebildete Polyol mindestens ein Fettsäurepolyol, ausgewählt aus 8,9,10- und 9,10,11-Trihydroxystearinsäure, und/oder mindestens ein Fettsäureesterpolyol mit mindestens einem 8,9,10- oder 9,10,11-Trihydroxystearinsäurerest ist.

7. Verfahren nach Anspruch 3, wobei Schritt (b) bei pH ≤ 7 erfolgt und das gebildete Polyol ein Fettsäurepolyol oder ein Fettsäureesterpolyol ist und das gebildete Polyol pro Doppelbindung der ursprünglichen ungesättigten Fettsäure oder pro Doppelbindung des ursprünglichen Fettsäureesters mehr als 2 Hydroxylgruppen aufweist.

8. Verfahren nach Anspruch 7, wobei Schritt (b) in Gegenwart einer Mischung aus Wasser und Perchlorsäure erfolgt.

9. Verfahren nach Anspruch 7 oder 8, wobei das gebildete Polyol mindestens eines, ausgewählt aus 8,9,10- und 9,10,11-Trihydroxystearinsäure, ist oder mindestens ein Fettsäurerest des gebildeten Polyols ein 8,9,10- oder 9,10,11-Trihydroxystearinsäurerest ist.

10. Verfahren nach einem der Ansprüche 1 bis 9, wobei das ungesättigte Fettsäurederivat ein Triglycerid ist.

11. Verfahren nach einem der Ansprüche 1 bis 10, wobei die ungesättigte Fettsäure Ölsäure ist oder der mindestens eine ungesättigte Fettsäurerest des Fettsäurederivats ein Ölsäurerest ist.

12. Verfahren nach einem der Ansprüche 1 bis 11, wobei die ungesättigte Fettsäure in Schritt (a) alleine oder im Gemisch mit einer oder mehreren unterschiedlichen gesättigten oder ungesättigten Fettsäuren vorliegt, oder das Fettsäurederivat, das mindestens einen ungesättigten Fettsäurerest mit einer oder mehreren Doppelbindungen aufweist, in Schritt (a) alleine oder im Gemisch mit einem oder mehreren unterschiedlichen gesättigten oder ungesättigten Fettsäurederivaten vorliegt oder wobei im Schritt (a) ein Gemisch von einem oder mehreren unterschiedlichen Fettsäuren und einem oder mehreren unterschiedlichen Fettsäurederivaten vorliegt, wobei mindestens eine Fettsäure oder ein Fettsäurederivat ungesättigt ist.

## Claims

1. A method for producing a polyol from an unsaturated fatty acid with one or more double bonds or a polyol from a fatty acid derivative, selected from fatty acid esters, fatty ethers, fatty alcohols, fatty acid amides, fatty acid chlorides, fatty amines, and fatty acid anhydrides, which has at least one unsaturated fatty acid residue with one or more double bonds, comprising the steps of
(a) peroxidizing an allyl unit of the unsaturated fatty acid or of the unsaturated fatty acid derivative, thus forming an allyl hydroperoxide unit, wherein the allyl hydroperoxide unit is subsequently converted into an epoxyalcohol unit, and
(b) opening the epoxy group of the resulting epoxyalcohol unit by means of hydrolysis, thus forming the polyol,
wherein step (a) occurs in the presence of a transition metal catalyst, the peroxidation of the allyl unit in step (a) is a photoperoxidation in the presence of a peroxidation means and a photosensitizer, wherein singlet oxygen is used as peroxidation means, and wherein the catalysis in step (a) occurs *in situ.*

2. The method according to claim 1, wherein the transition metal catalyst is a transition metal catalyst selected from a group consisting of titanium isopropoxide, vanadyl acetylacetonate and molybdenum oxyacetylacetonate.

3. The method according to claim 1 or 2, wherein the fatty acid derivative is a fatty acid ester.

4. The method according to claim 3, wherein step (b) occurs at a pH value of >7 and the formed polyol is a fatty acid polyol and/or a fatty acid ester polyol, and wherein the formed polyol has more than 2 hydroxyl groups per double bond of the original unsaturated fatty acid or per double bond of the corresponding fatty acid residue of the original fatty acid ester.

5. The method according to claim 4, wherein step (b) occurs in the presence of a base, selected from a group consisting of caustic soda (NaOH), caustic potash (KOH), ammonia (NH₃), calcium hydroxide (Ca(OH)₂), sodium carbonate (Na₂CO₃), and potassium carbonate (K₂CO₃).

6. The method according to claim 4 or 5, wherein the formed polyol is at least a fatty acid polyol, selected from 8,9,10- and 9,10,11-trihydroxy-stearic acid, and/or at least one fatty acid polyol with at least an 8,9,10- and 9,10,11-trihydroxy-stearic acid residue.

7. The method according to claim 3, wherein step (b) occurs at a pH value of ≤ 7 and the formed polyol is a fatty acid polyol or a fatty acid ester polyol, and wherein the formed polyol has more than 2 hydroxyl groups per double bond of the original unsaturated fatty acid or per double bond of the original fatty acid ester.

8. The method according to claim 7, wherein step (b) occurs in the presence of a mixture of water and perchloric acid.

9. The method according to claim 7 and 8, wherein the formed polyol is at least one, selected from 8,9,10- and 9,10,11-trihydroxy-stearic acid, or wherein at least one fatty acid residue of the formed polyol is an 8,9,10- and 9,10,11-trihydroxy-stearic acid residue.

10. The method according to any of the claims 1 to 9, wherein the unsaturated fatty acid derivative is a triglyceride.

11. The method according to any of the claims 1 to 10, wherein the unsaturated fatty acid is oleic acid, or wherein the at least one unsaturated fatty acid residue of the fatty acid derivative is an oleic acid residue.

12. The method according to any of the claims 1 to 11, wherein the unsaturated fatty acid in step (a) is present alone or as a mixture with one or more different saturated or unsaturated fatty acids, or wherein the fatty acid derivative having at least one unsaturated fatty acid residue with one or more double bonds in step (a) is present alone or as a mixture with one or more different saturated or unsaturated fatty acid derivates, or wherein in step (a), a mixture of one or more different fatty acids and one or more different fatty acid derivatives is present, wherein at least one fatty acid or one fatty acid derivative is unsaturated.

## Revendications

1. Procédé de préparation d'un polyol à partir d'un acide gras insaturé ayant une ou plusieurs liaisons doubles ou d'un polyol à partir d'un dérivé d'acide gras, sélectionné parmi un ester d'acide gras, un éther gras, des alcools gras, des amides d'acide gras, des chlorures d'acide gras, des amines grasses et des anhydrides d'acide gras, lequel présente au moins un résidu d'acide gras insaturé ayant une ou plusieurs liaisons doubles, comprenant les étapes suivantes:
(a) peroxydation d'un motif allyle de l'acide gras insaturé ou du dérivé d'acide gras insaturé avec formation d'un motif hydroperoxyde d'allyle, le motif hydroperoxyde d'allyle étant ensuite transformé en un motif époxyalcool, et
(b) ouverture du groupement époxy du motif époxyalcool apparu, par hydrolyse avec formation du polyol,
l'étape (a) étant effectuée en présence d'un catalyseur de métal de transition, la peroxydation du motif allyle à l'étape (a) étant une photoperoxydation en présence d'un agent de peroxydation et d'un photosensibilisateur, de l'oxygène singulet étant utilisé comme agent de peroxydation et la catalyse étant effectuée *in situ* à
l'étape (a).

2. Procédé selon la revendication 1, dans lequel le catalyseur de métal de transition est un catalyseur de métal de transition sélectionné parmi le groupe consistant en isopropoxyde de titane, oxyacétylacétonate de vanadium et oxyacétylacétonate de molybdène.

3. Procédé selon la revendication 1 ou 2, dans lequel le dérivé d'acide gras est un ester d'acide gras.

4. Procédé selon la revendication 3, dans lequel l'étape (b) est effectée à pH > 7 et le polyol formé est un polyol d'acide gras et/ou un polyol d'ester d'acide gras et le polyol formé présente plus de 2 groupements hydroxyle par double liaison de l'acide gras insaturé de départ ou par double liaison du résidu d'acide gras correspondant de l'ester d'acide gras de départ.

5. Procédé selon la revendication 4, dans lequel l'étape (b) est effectuée en présence d'une base, sélectionnée parmi le groupe consistant en soude caustique (NaOH), lessive de potasse (KOH), ammoniac (NH₃), hydroxyde de calcium (Ca(OH)₂), carbonate de sodium (Na₂CO₃) et carbonate de potassium (K₂CO₃).

6. Procédé selon la revendication 4 ou 5, dans lequel le polyol formé est au moins un polyol d'acide gras sélectionné parmi l'acide 8,9,10-trihydroxystéarique et 9,10,11-trihydroxystéarique, et/ou est au moins un polyol d'ester d'acide gras ayant au moins un résidu d'acide 8,9,10-trihydroxystéarique ou 9,10,11-trihydroxystéarique.

7. Procédé selon la revendication 3, dans lequel l'étape (b) est effectuée à pH ≤ 7 et le polyol formé est un polyol d'acide gras ou un polyol d'ester d'acide gras et le polyol formé présente plus de 2 groupements hydroxyle par double liaison de l'acide gras insaturé de départ ou par liaison double de l'ester d'acide gras de départ.

8. Procédé selon la revendication 7, dans lequel l'étape (b) est effectuée en présence d'un mélange d'eau et d'acide perchlorique.

9. Procédé selon la revendication 7 ou 8, dans lequel le polyol formé est un polyol d'au moins l'un parmi l'acide 8,9,10-trihydroxystéarique et 9,10,11-trihydroxystéarique, ou au moins un résidu d'acide gras du polyol formé est un résidu d'acide 8,9,10-trihydroxystéarique ou 9,10,11-trihydroxystéarique.

10. Procédé selon l'une des revendications 1 à 9, dans lequel le dérivé d'acide gras insaturé est un triglycéride.

11. Procédé selon l'une des revendications 1 à 10, dans lequel l'acide gras insaturé est l'acide oléique ou ledit au moins un résidu d'acide gras insaturé du dérivé d'acide gras est un résidu d'acide oléique.

12. Procédé selon l'une des revendications 1 à 11, dans lequel l'acide gras insaturé est présent à l'étape (a) seul ou en mélange avec un ou plusieurs acides gras saturés ou insaturés différents, ou le dérivé d'acide gras, qui présente au moins un résidu d'acide gras insaturé avec une ou plusieurs doubles liaisons, est présent à l'étape (a) seul ou en mélange avec un ou plusieurs dérivés d'acide gras saturés ou insaturés différents, ou à l'étape (a), un mélange d'un ou de plusieurs acides gras différents avec un ou plusieurs dérivés d'acide gras différents est présent, au moins un acide gras ou un dérivé d'acide gras étant insaturé.
